# EUROPEAN PATENT APPLICATION

(11) **EP 1 050 281 A1**
(43) Date of publication of application: **08.11.2000**
(21) Application number: 99201413.4
(22) Date of filing: 05.05.1999
(51) Int. Cl.: A61B 19/04

(54) **Use of modified starch as donning powder for surgical gloves**

(71) Applicant: Budev Medical B.V., 5271 AT St. Michielsgestel (NL)
(72) Inventor: Paping, Max Gregor, 5271 AT St. Michielsgestel (NL); Jeekel, Johannes, 3062 HM Rotterdam (NL)
(74) Representative: Van Someren, Petronella F. H. M.

(57) **Abstract**

The present invention relates to the use of modified starch as donning powder for surgical gloves, wherein the used starch is a granular, low crystalline, preferably a non-crystalline starch. The invention further relates to a surgical glove provided with said modified starch as donning powder at least on the surface contacting the skin of the user. By the use of the modified starch as a donning powder for surgical gloves, granuloma and ashesion formation due to starch contamination of the body is reduced.

## Description

The present invention relates to the use of modified starch as donning powder for surgical gloves. The invention further relates to a surgical glove provided with said modified starch as donning powder.

In the process of making surgical or examination gloves a mould of glass or ceramic is dipped in a concentrate of liquid natural rubber latex. After drying, the resulting rubber product remains a little sticky. In order to reduce this stickiness generally a starch powder is applied to the gloves after manufacturing.

Starch (mostly corn starch), which absorbs humidity, thus is the main constituent of glove powder. When used in surgery, it is possible that some of this corn starch powder is left behind in the patient's wound. This would not be a problem if the starch were completely absorbed by the body. However, it has been shown that residual starch can lead to the formation of granulomas and adhesions. These granulomas are caused by foreign particles which cannot be broken down in the body and form adhesions. When the damaged tissue is investigated with an optical microscope with crossed polarisers a Maltese cross is observed, typical for the presence of starch granules.

To prevent the formation of starch powder granulomas after operation it is known to remove all traces of the starch powder from the glove. However, in order to obtain totally powder free gloves the gloves have to be rinsed intensively with chemical compounds, which is both time-consuming and expensive.

It is also known to use non-powdered gloves in order to reduce the incidence of starch granulomas and adhesions. Several non-powdered gloves are on the market, and the lubrication of these gloves is obtained by a variety of methods, ranging from hydrogels to multilayer systems. However, these non-powdered gloves are far more expensive (about 3 times) than the powdered ones. In addition, non-powdered gloves are thicker and thus less comfortable to wear than powdered gloves. They are more slippery, more difficult to don (the hands must be totally dry) and have a worse grip on the instruments.

The object of the present invention is therefore to provide a donning powder for surgical gloves which is easily absorbed by body tissues and thus does not give rise to granuloma formation and adhesions when introduced into the body.

In order to achieve this, the present invention provides the use of a modified starch as donning powder for surgical gloves. According to the present invention it has thus been found that by the decrease of crystallinity of the modified starch according to the invention granuloma and adhesion formation due to starch contamination of body tissues can be reduced.

Initially, surgical gloves were sterilized by means of autoclaving. The replacement of this technique by gamma sterilization resulted in a dramatic increase of case reports of starch granulomas. It has been shown that autoclaved starch was almost completely absorbed from the peritoneal cavity of a rat within a period of 48 h, whereas irradiated starch was still not fully absorbed after 70 days. Scanning electron microscopy indicated that autoclaved starch showed pitting and cracking of the granule surface, while irradiated starch showed a smooth surface. It was therefore concluded that autoclaving damaged the starch in such a way that rapid absorption occurs.

Native starch is normally deposited in roots, tubers, grains etc, as semi-crystalline granules. It is known from the literature that the amorphous (non-crystalline) parts of the starch granules are easily attacked by the amylase enzymes which are present in saliva and blood. In contrast, the crystalline parts of the granule, which are more ordered and dense, are not very sensitive to enzymatic attack. For this reason, the semi-crystalline starch granules, if introduced in the human or animal body, are likewise not sensitive to enzymes, and are therefore not easily absorbed by the body tissues.

In the research that led to the present invention it has been found that in order to be suitable as donning powder for rubber gloves, the starch powder should have a suitable particle size (<50 µm). Starch having larger particles, like thermoplastic starch pellets, should be ground which will increase the price of the powder. In addition, the low- or non-crystalline starch should be spherical or oval shaped in order to preserve the lubrication properties. This means that the best shape is the granular form of unmodified starch.

According to a preferred embodiment of the present invention, the modified starch thus is a granular, low crystalline, preferably non-crystalline, starch. The granular, low-crystalline modified starch preferably has a so-called V-type crystal structure.

Methods for reducing the crystallinity of starch are known, based on the gelatinisation of starch with water or glycerol at elevated temperatures, or by increasing the pH by using NaOH. Such methods for the preparation of granular non-crystalline starch are for example described in US 3,617,383, US 4,465,702, and US 4,634,596, which relate to a method for the preparation of cold water swelling starches. This method is based on mixing the granular, crystalline starch with water and a non-solvent for the starch, such as methanol or ethanol, and heating the slurry to temperatures between 140 and 180°C at elevated pressures. An alternative method has been described in US 5,037,929 wherein the alcohol is substituted by a polyhydric alcohol, like propanediol or glycerol. The temperature can thus be reduced to 100-120°C and an atmospheric pressure can be applied. In US 5,057,157 granular cold water swelling starch is obtained by alcoholic/alkali treatments at ambient temperatures and pressures. These procedures result in the formation of V-type crystals or to an amorphous starch structure. The application of modified starch as a donning powder for rubber gloves has, however, not been described before.

In the research that led to the present invention five different types of starch were modified using a heat and/or alkali treatment in order to reduce the crystallinity in the granules. Two of the used modification methods were already described in the literature. In a third method only water and a sodium hydroxide solution was used. These methods are further described in Example 1.

The modified starches were characterized by optical microscopy with crossed polarizers for the measurement of birefringence (indicating the presence or absence of crystallinity). In addition, the amount and type of crystallinity was determined by X-ray diffraction. It was found that all three modification methods reduced the crystallinity, or even completely eliminated the crystalline structure of the starch granules.

According to a preferred embodiment of the present invention the birefringence of the modified starch is less than 30%, preferably less than 20%, more preferably less than 10%, and most preferably less than 5% of native starch.

Furthermore, it has been found that the starch powder should not be completely soluble in cold water, because this would cause the gloves to become too sticky and reduce the wearing comfort. For these reasons, the use of thermoplastic starch or lower molecular carbohydrates like maltodextrines is eliminated. According to a preferred embodiment of the invention preferably less than 75% of the modified starch is soluble in cold water.

Preferably, the modified starch according to the present invention is derived from native potato starch, native corn starch, native rice starch, or waxy corn starch.

The modified starch of the present invention is preferably used as a donning powder for rubber latex gloves, so called surgical gloves. Such gloves may however also be used for various other medical and non-medical applications.

The invention further relates to a surgical glove provided with modified starch as a donning powder at least on the surface of the glove to be contacting the skin of the user. To provide a surgical glove with the modified starch, different known methods of powdering the gloves may be used.

The invention will further be illustrated by the following example and figure.

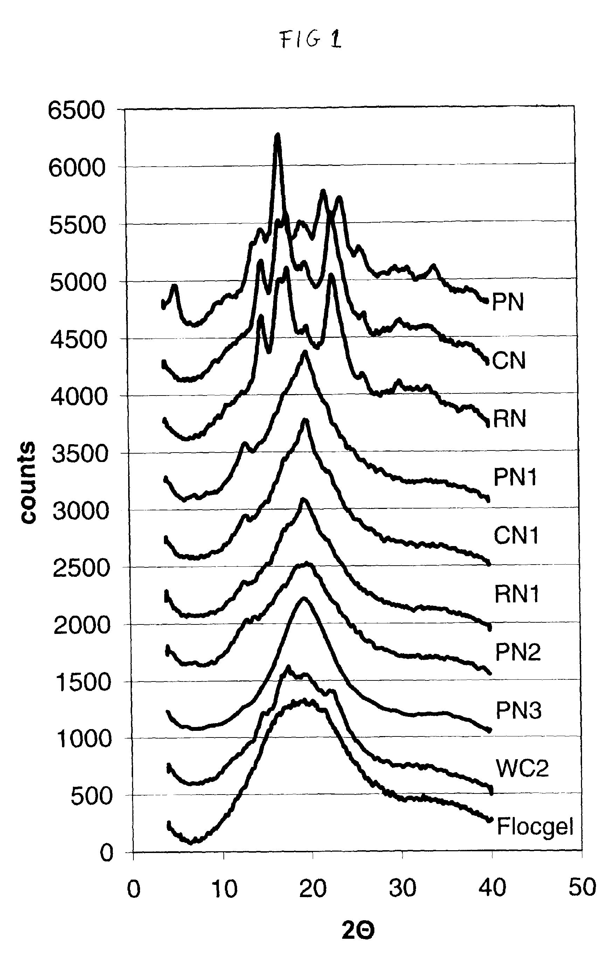

In figure 1 the results of the X-ray diffraction measurements of the modified starches are visualized.

### EXAMPLE

### EXAMPLE 1

### Preparation of modified starch powder

The starch preparations which were used were native potato starch (PN), native corn starch (CN) and native rice starch (RN). Native means that the starches have not undergone any modification prior to use. One waxy starch was used, viz. waxy corn (WC). This starch contains a high amount of amylopectin (>99%) and hardly any amylose. A pregelatinised starch (flocgel) was also incorporated in the measurements. This starch was ground after modification in order to obtain small particles possibly suitable for glove powdering.

As solvents water, glycerol and denaturated ethanol were used. A 1M solution of sodium hydroxide in water was used to increase the pH and provoke gelatinisation of some of the starches.

Three different methods for the preparation of the modified starch were used:
1. In a first method 10 g starch was added to a mixture of 38.8 g glycerol and 11.6 g water in an Erlenmeyer flask. The Erlenmeyer flask was put into a paraffin bath and heated to 130-140°C. The mixture was homogenised by a magnetic stirrer. After about 5 min, the viscosity of the slurry increased, at which time the Erlenmeyer was retrieved from the paraffin bath and cooled down to 100°C and about 100ml of ethanol or of an ethanol/glycerol (1:4) mixture was added. The slurry was stirred until a homogeneous mixture was obtained. This mixture was suction filtered, after which the solid mass was redispersed in ethanol in order to remove the water. This was repeated. The powder thus obtained was dried at 50°C. This method has been described in US 5,037,929.
2. In a second method the same amounts of glycerol and starch were mixed with 10 g 1M NaOH solution. The paraffin bath was set on 120°C, which resulted in a temperature of the slurry of 100°C. After 5 min, the slurry became more viscous and the Erlenmeyer flask was removed from the heat source. Hydrochloric acid was added in order to neutralise the mixture. The viscous paste was washed with 100 ml of ethanol or ethanol/glycerol (1:4) and suction filtered.
   Subsequently, the powder was washed twice with ethanol and dried at 50°C.
3. In the third method 50 g of water was mixed with 5 g starch in an Erlenmeyer flask. A 1M NaOH solution was added slowly into the mixture to ensure an overall concentration of 0.2M NaOH (=13g 1M NaOH). After the viscosity had increased, 100 ml ethanol was added to the slurry. This mixture was stirred and homogenised, and hydrochloric acid was added to neutralise the mixture. The powder obtained after suction filtration was immersed twice in ethanol and dried at 50°C.

The powder which was obtained by these methods was sieved over a 90 µm sieve.

### Characterization of the modified starch powder

The powders were characterised by their behaviour in cold water and examined under an optical microscope with crossed polarisers (Zeiss Axioplan). Furthermore, the amount and type of crystallinity was determined using X-ray diffraction (Philips PW3710).

The soluble fraction of the powder was obtained by mixing 0.1 g of modified starch with 5 g of cold water in a small polystyrene container. The mixture was stirred and put aside at room temperature for 24 hours, and stirred every hour for the first 5 hours. After 24 hours a layer of gelled and unmodified particles sedimented on the bottom of the container. This layer was separated from the clear liquid above, dried in a vacuum oven at 50°C and weighed.

Since all the granulomas formed after starch contamination of body tissue showed a Maltese cross, the modified powder was also subjected to a birefringence test. The amount of particles which still showed birefringence, even after modification, was determined using an optical microscope. The modified starch was immersed in water and put between crossed polarisers. The unchanged particles showed a yellow and blue cross, whereas of the modified particles only the contours were visible. The absence of the Maltese crosses indicated a loss of original crystallinity.

X-ray diffraction was used in order to obtain information about the amount and type of residual crystallinity. Radiation from a Cu K-α source was reflected by the sample and detected by a detector, moving from 2θ = 4° to 2θ = 40°. The various types of crystal structures were distinguished by their peak positions. The double helical amylopectin structures are indicated by A, B and C crystallinity, and the single helical amylose by V crystals.

Specimens of non-crosslinked natural rubber were dusted with the modified starch in order to determine whether the powder is applicable as a glove lubricant or not. The dusted rubber was tested for comfort and lubricity. The surface was wetted with cold water and tested for stickiness. Powder, which becomes very sticky is not very suitable as a lubricant.

The results of the sample preparation and material characterisation are listed in table 1. From this table, it can be concluded that the degree of solubility and amount of residual birefringence (birefr.) depends on the modification method used. The highest fraction of starch soluble in cold water is derived by a treatment with a high concentration of NaOH. The source of starch does not play a very important role. However, the waxy type, having a high amylopectin fraction, is less sensitive to the modification. The waxy starch was used in order to prevent recrystallisation of the amylose after gelatinisation of the original starch granules.

**Table 1**

| Starch^{a} | Method | solv.^{b} | solubility % | Birefr . % | Cryst. type^{c} | comfort^{d} wet behaviour |
|---|---|---|---|---|---|---|
| PN | - | - | 0 | 100 | B | +, N |
| CN | - | - | 0 | 100 | A | +, N |
| RN | - | - | 0 | 100 | A | +, N |
| PN1 | 1 | eth. | 45-50 | 5-10 | V | +/-, S |
| CN1 | 1 | eth. | 35-40 | 5-10 | V | +, N |
| RN1 | 1 | eth. | 35-40 | 1-5 | V | +, N |
| PN2 | 2 | eth. | 40-45 | 5-10 | V/Am | +, S |
| PN3 | 3 | eth. | 75-80 | 1-5 | Am | +, S |
| WC2 | 2 | eth./ glyc. | 60-65 | 20-30 | A/Am | +, N |
| Flocgel | - | - | 100 | 0 | Am | -, V |

| | | | | | | |
|---|---|---|---|---|---|---|
| ^{a} PN: Native potato; CN: Native corn; RN: Native rice; WC: Waxy corn; high amylopectin content; Flocgel: Gelatinised and ground starch; | | | | | | |
| ^{b} Eth: Ethanol, Glyc: Glycerol | | | | | | |
| ^{c} Am: Amorphous; | | | | | | |
| ^{d} N: Not sticky; S: Slightly sticky; V: Very sticky; +: Good comfort; +/-: Reasonable comfort; - : Bad comfort. | | | | | | |

In figure 1 the results from the X-ray measurements are shown. In this figure, the different curves are vertically shifted 500 counts. It can be seen that the crystallinity of the native starch sources (PN, CN, RN) is high and can be divided into an A and B type crystallinity. The potato, corn and rice starches, modified according to method 1 (PN1, CN1, RN1) all gave a similar X-ray pattern, viz. V-type crystallinity. This is indicated by the peaks at 2θ ≈ 14 and 20°. The two potato starches treated with NaOH (PN2 and PN3) showed a very low crystallinity. The diffraction pattern for an amorphous starch structure was visible for PN3 (obtained by method 3). Flocgel showed an amorphous X-ray pattern indicating the absence of residual crystallinity. Finally, the crystallinity of the waxy starch was reduced considerably. It was clear that no V-type crystallinity was formed, since the peaks at 2θ ≈ 14 and 20° were absent.

The behaviour of the powder when applied to the sticky surface of non-crosslinked natural rubber was diverse. The granular starches reduced the stickiness of the gloves. The results of the potato starch were slightly less smooth, due to the larger granule size. Dusting the rubber surface with Flocgel did not result in a smooth surface, because the particles obtained by grinding the gelatinised starch were too coarse.

After wetting the dusted surfaces the stickiness was again tested. The Flocgel became very sticky, because the powder dissolved almost completely in cold water. The modified potato starches (PN1, PN2 and PN3) showed a slight stickiness. The waxy starch and corn and rice starch did not show an enhanced stickiness. Comparing these findings to the results of the solubility measurements, it is obvious that the amount of soluble material in the dusting powder has a large influence on the wet behaviour. The solubility, and thus the stickiness, can be reduced by crosslinking the powder before or after modification. In this way the soluble chains are incorporated in the granules.

## Claims

1. Use of modified starch as donning powder for surgical gloves.

2. Use according to claim 1 **characterized in that** the modified starch is a granular, low crystalline, preferably non-crystalline, starch.

3. Use according to claim 2 **characterized in that** the low-cristalline starch has a V-type crystal structure.

4. Use according to claim 1, 2 or 3 **characterized in that** the birefringence of the modified starch is less than 30%, preferably less than 20%, more preferably less than 10%, and most preferably less than 5% of native starch.

5. Use according to any of the preceding claims 1-4 **characterized in that** less than 75% of the modified starch is soluble in cold water.

6. Use according to any of the preceding claims 1-5 **characterized in that** the modified starch is modified potato starch, modified corn starch, modified rice starch, or modified waxy corn starch.

7. Surgical glove provided with modified starch as a donning powder at least on the surface of the glove to be contacting the skin of the user.

8. Surgical glove according to claim 7 **characterized in that** the modified starch is a granular, low crystalline, preferably non-crystalline, starch.

9. Surgical glove according to claim 8 **characterized in that** the low-cristalline starch has a V-type crystal structure.

10. Surgical glove according to claim 7, 8 or 9 **characterized in that** the birefringence of the modified starch is less than 30%, preferably less than 20%, more preferably less than 10%, and most preferably less than 5% of native starch.

11. Surgical glove according to any of the claims 7-10 **characterized in that** less than 75% of the modified starch is soluble in cold water.

12. Surgical glove according to any of the preceding claims 7-11 **characterized in that** the modified starch is preferably modified potato starch, modified corn starch, modified rice starch, or modified waxy corn starch.
